# EUROPEAN PATENT APPLICATION

(11) **EP 4 843 544 A1**
(43) Date of publication of application: **25.11.2026**
(21) Application number: 24917652.0
(22) Date of filing: 16.01.2024
(51) Int. Cl.: A61F 9/007, A61B 17/34

(54) **OPHTHALMIC PUNCTURE DEVICE**

(71) Applicant: Belle Healthcare Technology Co., Ltd., Suzhou, Jiangsu 215163 (CN)
(72) Inventor: ZHAO, An, Suzhou, Jiangsu 215163 (CN); LI, Zhongze, Suzhou, Jiangsu 215163 (CN); LI, Jumin, Suzhou, Jiangsu 215163 (CN)
(74) Representative: Rau, Schneck & Hübner Patentanwälte Rechtsanwälte PartGmbB
(86) International application number: PCT/CN2024/072507
(87) International publication number: WO 2025/152025

(57) **Abstract**

An ophthalmic puncture device (100) includes: a handle (10); a puncture blade tip (20) provided at one end of the handle (10); a protective sleeve (30) provided inside the handle (10) and movable between a first position and a second position along an axial direction of the handle (10), wherein the puncture blade tip (20) is located inside the protective sleeve (30) when the protective sleeve (30) is located at the first position, and is exposed from the protective sleeve (30) when the protective sleeve (30) is located at the second position; a slide key (40) connected to the protective sleeve (30) and movable along the axial direction of the handle (10) to drive the protective sleeve (30); and a puncture cannula (50) detachably sleeved over the puncture blade tip (20), wherein the protective sleeve (30) cooperates with the puncture cannula (50) to push the puncture cannula (50) out of the puncture blade tip (20) when the protective sleeve (30) moves from the second position to the first position.

## Description

### TECHNICAL FIELD

The present disclosure relates to the technical field of ophthalmic surgery, and particularly to an ophthalmic puncture device.

### BACKGROUND

In ophthalmic microsurgery, a surgeon may need to insert and withdraw surgical instruments multiple times, or insert and withdraw a plurality of surgical instruments. If a surgical instrument directly contacts an incision site, the insertion, operation and withdrawal of the surgical instrument may cause trauma to an eye. To meet the needs of providing simple insertion and withdrawal of surgical instruments, reducing the possibility of trauma at the incision site, and promoting healing, the surgeon may insert one or more puncture cannulas into the eye, each puncture cannula serving as an access passage or access port for the surgical instrument to enter the eye.

In fundus surgery, it is generally required to first place the above puncture cannula on an ocular surface; before puncturing, a scale ruler is used to appropriately mark a puncture position, and then puncturing is performed; after the puncturing is completed, a puncture blade is withdrawn, and the puncture cannula is left on the ocular surface to form a passage for operation of the surgical instrument.

At present, after the puncturing is completed, it is necessary to use forceps to assist in clamping the puncture cannula so as to withdraw the surgical instrument; this process requires two-hand cooperation or multi-person cooperation to complete, which not only affects operation efficiency, but also increases surgical risks.

### SUMMARY

The present disclosure provides a new technical solution of an ophthalmic puncture device, which can at least solve the problems of low assembly efficiency of puncture cannulas and high surgical risks in the prior art.

The ophthalmic puncture device according to an embodiment of the present disclosure comprises: a handle; a puncture blade tip provided at one end of the handle; a protective sleeve provided inside the handle and movable between a first position and a second position along an axial direction of the handle, wherein the puncture blade tip is located inside the protective sleeve when the protective sleeve is located at the first position, and is exposed from the protective sleeve when the protective sleeve is located at the second position; a slide key connected to the protective sleeve and movable along the axial direction of the handle to drive the protective sleeve; a puncture cannula detachably sleeved over the puncture blade tip, wherein the protective sleeve cooperates with the puncture cannula to push the puncture cannula out of the puncture blade tip when the protective sleeve moves from the second position to the first position.

Optionally, the protective sleeve comprises: a sleeve movable along the axial direction of the handle, one end of the sleeve being provided with a mounting recess, a part of the slide key being connected to the mounting recess; a limiting member provided on the sleeve, the limiting member abutting against the puncture cannula when the protective sleeve is located at the second position.

Optionally, the limiting member comprises: a connecting portion connected to the sleeve; a limiting portion movable relative to the connecting portion between a retracted state and an expanded state, wherein when the limiting portion is in the retracted state, the limiting portion avoids the puncture cannula, and the protective sleeve is movable from the first position to the second position; when the limiting portion is in the expanded state, the limiting portion is located on a movement path of the puncture cannula, and the limiting portion is separated from the puncture cannula or stops against the puncture cannula.

Optionally, the connecting portion is formed as an annular structure extending along a circumferential direction of the sleeve, and the limiting portion is provided on an inner circumference of the connecting portion and extends along a circumferential direction of the connecting portion.

Optionally, the limiting portion is formed as an elastic body provided on the connecting portion, and is deformable to switch between the retracted state and the expanded state.

Optionally, the limiting portion is formed as an elastic body extending obliquely relative to an axis of the puncture cannula, and in a direction in which the protective sleeve moves from the first position to the second position, the limiting portion gradually slopes away from the axis of the puncture cannula.

Optionally, the connecting portion comprises: a body portion connected to the sleeve; a first bent portion formed as a C-shaped structure, a first end of the first bent portion being connected to the body portion; a second bent portion formed as a C-shaped structure with an opening direction opposite to an opening direction of the first bent portion, a first end of the second bent portion being connected to a second end of the first bent portion, the limiting portion being connected to a second end of the second bent portion.

Optionally, an end of the sleeve is provided with a positioning groove where the body portion and the first bent portion are provided.

Optionally, the limiting member is an integrally formed metal sheet or metal wire.

Optionally, the handle is provided with a guide slot extending along an axial direction thereof, the slide key is movably provided in the guide slot, and a part of the slide key passes through the guide slot to be connected to the protective sleeve.

In the ophthalmic puncture device according to the embodiment of the present disclosure, by providing a movable protective sleeve inside the handle, the protective sleeve can push the puncture cannula to disengage from the puncture blade tip in the process of the protective sleeve moving from the second position to the first position, without additional clamping assistance by forceps, such that the disengagement of the puncture cannula can be completed by one-hand operation, so as to realize the withdrawal action of the puncture blade tip and effectively improve surgical efficiency; moreover, the one-hand operation of withdrawing the puncture blade tip from the puncture cannula can reduce surgical risks and improve safety.

Other features and advantages of the present disclosure will become apparent from the following detailed description of exemplary embodiments of the present disclosure with reference to the accompanying drawings.

### BRIEF DESCRIPTION OF THE DRAWINGS

The accompanying drawings, which are incorporated in the description and constitute a part of the description, illustrate embodiments of the present disclosure and, together with the description thereof, serve to explain the principles of the present disclosure.
FIG. 1 is a structural schematic view of an ophthalmic puncture device according to one embodiment of the present disclosure;
FIG. 2 is a side view of an ophthalmic puncture device according to one embodiment of the present disclosure;
FIG. 3 is a partial cross-sectional view of an ophthalmic puncture device according to one embodiment of the present disclosure;
FIG. 4 is a schematic view of one state of the limiting portion of an ophthalmic puncture device according to one embodiment of the present disclosure;
FIG. 5 is a schematic view of another state of the limiting portion of an ophthalmic puncture device according to one embodiment of the present disclosure;
FIG. 6 is a schematic view of yet another state of the limiting portion of an ophthalmic puncture device according to one embodiment of the present disclosure.

### Description of reference signs:

ophthalmic puncture device 100;
handle 10; guide slot 11;
puncture blade tip 20;
protective sleeve 30; sleeve 31; mounting recess 311; positioning groove 312; limiting member 32; connecting portion 321; body portion 3211; first bent portion 3212; second bent portion 3213; limiting portion 322;
slide key 40;
puncture cannula 50.

### DETAILED DESCRIPTION

Various exemplary embodiments of the present disclosure will now be described in detail with reference to the accompanying drawings. It is to be noted that unless otherwise specified, the scope of the present disclosure is not limited to relative arrangements, numerical expressions and values of components and steps as illustrated in the embodiments.

Description to at least one exemplary embodiment is for illustrative purpose only, and in no way implies any restriction on the present disclosure or application or use thereof.

Techniques, methods and devices known to those skilled in the prior art may not be discussed in detail; however, such techniques, methods and devices shall be regarded as part of the description where appropriate.

In all the examples illustrated and discussed herein, any specific value shall be interpreted as illustrative rather than restrictive. Different values may be available for alternative examples of the exemplary embodiments.

It is to be noted that similar reference numbers and alphabetical letters represent similar items in the accompanying drawings. In the case that a certain item is identified in a drawing, further reference thereof may be omitted in the subsequent drawings.

The ophthalmic puncture device 100 according to the embodiment of the present disclosure is specifically described below with reference to the accompanying drawings.

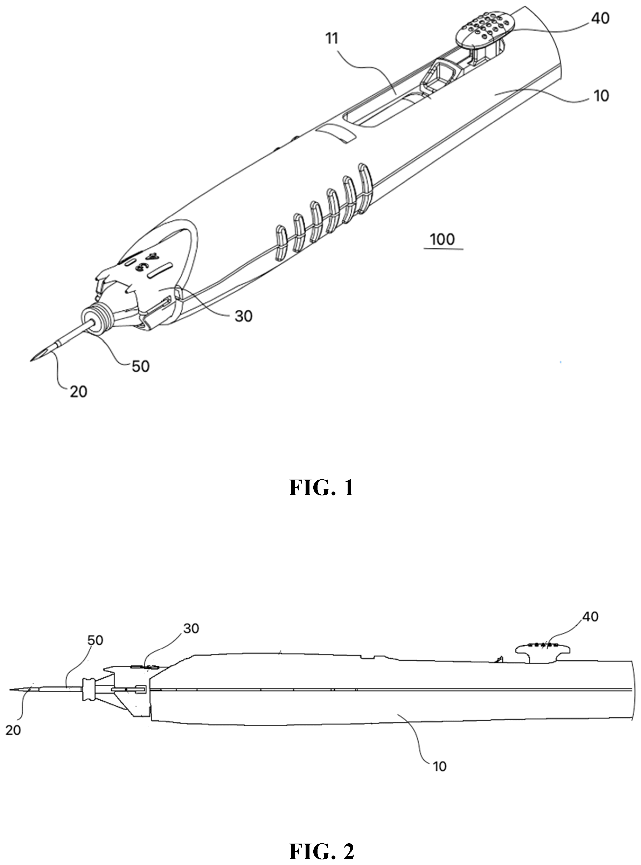

As shown in FIGS. 1 to 6, the ophthalmic puncture device 100 according to the embodiment of the present disclosure comprises: a handle 10, a puncture blade tip 20, a protective sleeve 30, a slide key 40 and a puncture cannula 50.

Specifically, the puncture blade tip 20 is provided at one end of the handle 10; the protective sleeve 30 is provided inside the handle 10 and is movable between a first position and a second position along an axial direction of the handle 10; when the protective sleeve 30 is located at the first position, the puncture blade tip 20 is located inside the protective sleeve 30; when the protective sleeve 30 is located at the second position, the puncture blade tip 20 is exposed from the protective sleeve 30; the slide key 40 is connected to the protective sleeve 30 and is movable along the axial direction of the handle 10 to drive the protective sleeve 30; the puncture cannula 50 is detachably sleeved over the puncture blade tip 20; the protective sleeve 30 cooperates with the puncture cannula 50 to push the puncture cannula 50 out of the puncture blade tip 20 when the protective sleeve 30 moves from the second position to the first position.

In other words, the ophthalmic puncture device 100 according to the embodiment of the present disclosure can be used in ophthalmic surgery. The ophthalmic puncture device 100 can push the puncture cannula 50 out of the puncture blade tip 20, so as to place the puncture cannula 50 on an ocular surface. The ophthalmic puncture device 100 according to the embodiment of the present disclosure is mainly composed of the handle 10, the puncture blade tip 20, the protective sleeve 30, the slide key 40 and the puncture cannula 50, wherein the handle 10 constitutes a body structure of the ophthalmic puncture device 100, and other components can be provided on the handle 10 directly or indirectly; the handle 10 is designed into a structure suitable for being held by a user, such as a columnar structure, so as to facilitate the user to operate and use the ophthalmic puncture device 100.

The puncture blade tip 20 is provided at one end of the handle, and can be used for puncturing an eyeball; the shape and structure of the puncture blade tip 20 can be reasonably adjusted according to specific surgical needs. The protective sleeve 30 is provided inside the handle 10 and is movable between the first position and the second position; when the protective sleeve 30 is located at the first position, one end of the protective sleeve 30 extends out of the handle 10 and can completely cover the puncture blade tip 20, so as to avoid risks caused by exposure of the puncture blade tip 20; when the protective sleeve 30 is located at the second position, one end of the protective sleeve 30 can be retracted into the handle 10, and the puncture blade tip 20 is exposed from the handle 10 and the protective sleeve 30; at this time, surgery can be performed by means of the puncture blade tip 20.

The slide key 40 is movably provided on the handle 10; a part of the slide key 40 is exposed from an outer surface of the handle 10 or is configured into a structure convenient for the user to push and pull, and another part of the slide key 40 can be connected to the protective sleeve 30, so that the protective sleeve 30 can be driven to move between the first position and the second position by sliding the slide key 40.

When the ophthalmic puncture device 100 according to the embodiment of the present disclosure is not in use, the protective sleeve 30 can be located at the first position or a position close to the first position, and the puncture blade tip 20 is located inside the protective sleeve 30; the puncture cannula 50 can be pre-sleeved over the puncture blade tip 20, a length of the puncture cannula 50 can be smaller than a length of the puncture blade tip 20, and a blade point of the puncture blade tip 20 can be exposed from the puncture cannula 50.

When it is required to use the ophthalmic puncture device 100, the slide key 40 can be pushed to move the protective sleeve 30 from the first position to the second position; after the blade point of the puncture blade tip 20 is exposed from the protective sleeve 30, surgery can be normally performed by means of the puncture blade tip 20.

When it is required to push the puncture cannula 50 out of the puncture blade tip 20, the protective sleeve 30 can be moved to the second position by pushing the slide key 40; when the protective sleeve 30 is located at the second position, a part of the protective sleeve 30 can cooperate with the puncture cannula 50, for example, can clamp the puncture cannula 50 or stop against the puncture cannula 50; then the protective sleeve 30 is pushed from the second position to the first position by means of the slide key 40; during the movement of the protective sleeve 30, the protective sleeve 30 can drive the puncture cannula 50 to move synchronously, so as to push the puncture cannula 50 out of the puncture blade tip 20; the pushed-out puncture cannula 50 can be placed on an ocular surface or other surfaces where the puncture cannula 50 needs to be reserved.

Accordingly, in the ophthalmic puncture device 100 according to the embodiment of the present disclosure, by providing the movable protective sleeve 30 inside the handle 10, the protective sleeve 30, driven by the slide key 40, can push the puncture cannula 50 to disengage from the puncture blade tip 20 in the process of moving from the second position to the first position, without additional clamping assistance by forceps; the disengagement of the puncture cannula 50 can be completed by one-hand operation, so as to realize the withdrawal action of the puncture blade tip 20 and effectively improve surgical efficiency; moreover, the one-hand operation of withdrawing the puncture blade tip 20 from the puncture cannula 50 can reduce surgical risks and improve safety.

According to one embodiment of the present disclosure, the protective sleeve 30 comprises: a sleeve 31 and a limiting member 32.

Specifically, the sleeve 31 is movable along the axial direction of the handle 10; one end of the sleeve 31 is provided with a mounting recess 311, and a part of the slide key 40 is connected to the mounting recess 311; the limiting member 32 is provided on the sleeve 31, and abuts against the puncture cannula 50 when the protective sleeve 30 is located at the second position.

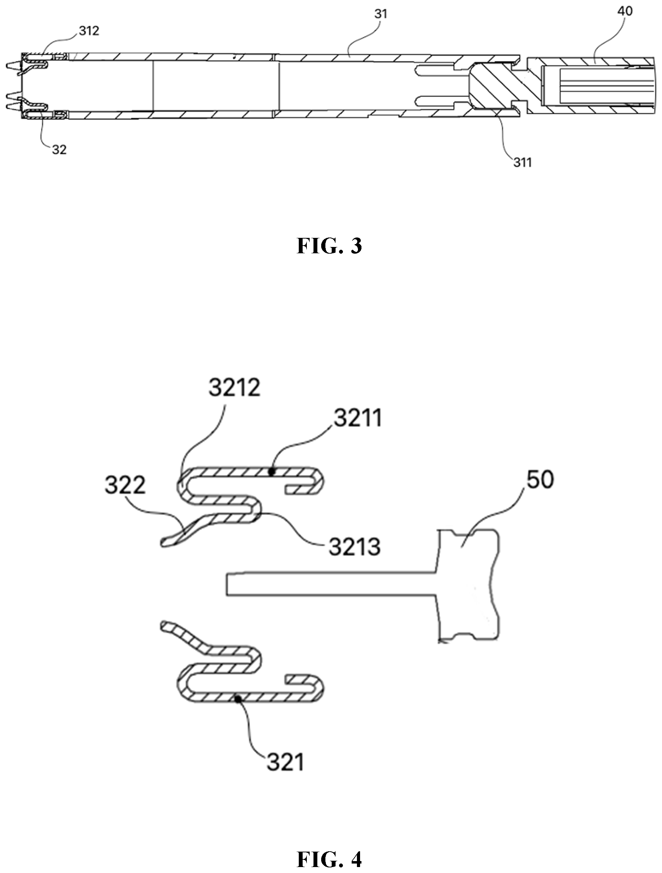

In other words, as shown in FIG. 3, the protective sleeve 30 is mainly composed of two parts: the sleeve 31 and the limiting member 32, wherein the sleeve 31 constitutes a body structure of the protective sleeve 30, and is provided inside the handle 10, and specifically can be formed into a columnar structure corresponding to the shape of the handle 10; one end of the sleeve 31 can extend out of or retract into the handle 10, so as to play a role of protecting the puncture blade tip 20 or exposing the puncture blade tip 20. The other end of the sleeve 31 is provided with a mounting recess 311, and a part of the slide key 40 extends into the handle 10 and is connected to the mounting recess 311, so as to realize synchronous movement of the slide key 40 and the protective sleeve 30.

The limiting member 32 is provided at one end of the sleeve 31 close to the puncture blade tip 20, and the limiting member 32 and the sleeve 31 can move synchronously; when the protective sleeve 30 moves to the second position, the limiting member 32 can abut against the puncture cannula 50; after the limiting member 32 abuts against the puncture cannula 50, the slide key 40 is pushed to cause the protective sleeve 30 to move from the second position to the first position, and the limiting member 32 can push the puncture cannula 50 to move synchronously, such that the puncture cannula 50 is pushed out of the puncture blade tip 20.

Accordingly, in the ophthalmic puncture device 100 according to the embodiment of the present disclosure, by providing the limiting member 32 on the sleeve 31, the limiting member 32 can abut against the puncture cannula 50 when the protective sleeve 30 is located at the second position, so that the puncture cannula 50 can be pushed out of the puncture blade tip 20 when the protective sleeve 30 moves from the second position to the first position; this structure is simple and feasible, and is convenient to operate.

In some specific implementations of the present disclosure, the limiting member 32 comprises: a connecting portion 321 and a limiting portion 322.

Wherein, the connecting portion 321 is connected to the sleeve 31; the limiting portion 322 is movable between a retracted state and an expanded state relative to the connecting portion 321; when the limiting portion 322 is in the retracted state, the limiting portion 322 avoids the puncture cannula 50, and the protective sleeve 30 is movable to move from the first position to the second position; when the limiting portion 322 is in the expanded state, the limiting portion 322 is located on a movement path of the puncture cannula 50, and is separated from the puncture cannula 50 or stops against the puncture cannula 50.

Specifically, as shown in FIGS. 3 to 6, the limiting member 32 according to the embodiment of the present disclosure is mainly composed of two parts: the connecting portion 321 and the limiting portion 322; the connecting portion 321 mainly plays a role of assembling the limiting member 32 on the sleeve 31, and the connecting portion 321 can be fixedly connected to the sleeve 31, or can be detachably connected to the sleeve 31 as required.

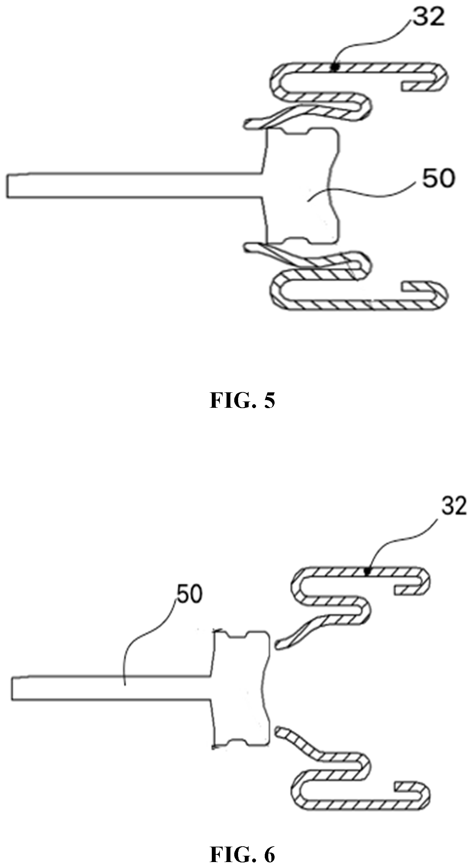

The limiting portion 322 is movably connected to the connecting portion 321, and can be switched between a retracted state and an expanded state. Wherein, when the limiting portion 322 is in the retracted state, as shown in FIG. 5, a part of the limiting portion 322 is retracted to form a space for avoiding the passage of the puncture cannula 50; at this time, the limiting portion 322 can move relative to the puncture cannula 50, so as not to affect the movement of the protective sleeve 30 from the first position to the second position. The retraction of the part of the limiting portion 322 may be realized by an external structure controlling the movement of the limiting portion 322, or may be realized by the puncture cannula 50 pressing the limiting portion 322 during the movement of the protective sleeve 30 from the first position to the second position, thereby causing the limiting portion 322 to deform.

When the limiting portion 322 is in the expanded state, a part of the limiting portion 322 is expanded. When the limiting portion 322 is in the expanded state, the protective sleeve 30 may be in the first position or the second position. When the protective sleeve 30 is in the first position, as shown in FIG. 4, both the puncture cannula 50 and the puncture blade tip 20 are located inside the protective sleeve, and the limiting portion 322 may be in a free state without contacting the puncture cannula 50. When the protective sleeve 30 is in the second position, as shown in FIG. 6, a part of the limiting portion 322 can stop against the puncture cannula 50, and when the protective sleeve 30 moves from the second position to the first position, the limiting portion 322 can drive the puncture cannula 50 to move together, so as to push the puncture cannula 50 out of the puncture blade tip 20.

Accordingly, by providing the retractable and expandable limiting portion 322 on the limiting member 32, the limiting portion 322 can avoid the puncture cannula 50 when the protective sleeve 30 moves from the first position to the second position, without affecting the normal movement of the protective sleeve 30, and can stop against the puncture cannula 50 when the protective sleeve 30 moves to the second position, so that the puncture cannula 50 can be pushed to disengage from the puncture blade tip 20 when the protective sleeve 30 moves from the second position to the first position. The limiting member 32 has a simple structure and is convenient to assemble and operate, so that the puncture cannula 50 can be pushed out easily by one hand, thereby greatly improving surgical efficiency and reducing surgical risks.

According to one embodiment of the present disclosure, the connecting portion 321 is formed as an annular structure extending along a circumferential direction of the sleeve 31, and the limiting portion 322 is provided on an inner circumference of the connecting portion 321 while extending along the circumferential direction of the connecting portion 321.

In other words, the limiting member 32 is integrally formed as an annular structure, the connecting portion 321 of the limiting member 32 extends along the circumferential direction of the sleeve 31, and the limiting portion 322 is provided on the entire circumference of the connecting portion 321. Wherein, it can be understood that the connecting portion 321 and the limiting portion 322 may also be provided in plurality, and the plurality of limiting portions 322 are distributed at intervals along the circumferential direction of the sleeve 31.

Accordingly, by providing the connecting portion 321 and the limiting portion 322 of such a structure, it can not only ensure the connection stability between the limiting member 32 and the sleeve 31, but also ensure that the limiting portion 322 can normally push the puncture cannula 50 to disengage from the puncture blade tip 20 when the sleeve 31 moves, which further improves product stability and safety.

In some specific implementations of the present disclosure, the limiting portion 322 is formed as an elastic body provided on the connecting portion 321, and the limiting portion 322 is deformable to switch between the retracted state and the expanded state.

Optionally, the limiting portion 322 is formed as an elastic body extending obliquely relative to an axis of the puncture cannula 50, and in a direction in which the protective sleeve 30 moves from the first position to the second position, the limiting portion 322 gradually slopes away from the axis of the puncture cannula 50.

Specifically, as shown in FIGS. 4 to 6, the limiting portion 322 may be formed as an elastic structure, and the elastic structure extends obliquely relative to the axis of the puncture cannula 50. The design of this structure enables the limiting portion 322 to be in a naturally expanded state under the action of its elastic force when the protective sleeve 30 is in the first position; when the protective sleeve 30 moves from the first position to the second position, the limiting portion 322 can be pressed by the puncture cannula 50 to move from the expanded state to the retracted state, and after the protective sleeve 30 moves to the second position, the limiting portion 322 can automatically expand; when the protective sleeve 30 moves from the second position to the first position, the limiting portion 322 can push the puncture cannula 50, so as to disengage the puncture cannula 50 from the puncture blade tip 20.

Accordingly, by designing the limiting portion 322 as an elastic body structure, the operation of the ophthalmic puncture device 100 can be further simplified, thereby further improving surgical efficiency.

According to one embodiment of the present disclosure, the connecting portion 321 comprises: a body portion 3211, a first bent portion 3212 and a second bent portion 3213.

Specifically, the body portion 3211 is connected to the sleeve 31; the first bent portion 3212 is formed as a C-shaped structure, and a first end of the first bent portion 3212 is connected to the body portion 3211; the second bent portion 3213 is formed as a C-shaped structure with an opening direction opposite to an opening direction of the first bent portion 3212, a first end of the second bent portion 3213 is connected to a second end of the first bent portion 3212, and the limiting portion 322 is connected to a second end of the second bent portion 3213.

In other words, as shown in FIG. 4, the connecting portion 321 may be composed of three parts: the body portion 3211, the first bent portion 3212 and the second bent portion 3213, which are connected in sequence. The body portion 3211 can fix the entire limiting member 32 to the sleeve 31; the first bent portion 3212 and the second bent portion 3213 are each formed as a C-shaped structure, that is, the first bent portion 3212 and the second bent portion 3213 can be connected to each other to form an S-shaped structure, and the limiting portion 322 is provided at an end of the second bent portion 3213.

Accordingly, the connecting portion 321 of such a structure can not only ensure the connection stability between the limiting portion 322 and the connecting portion 321, but also endow the limiting portion 322 with excellent elasticity, so as to realize free switching between the retracted state and the expanded state.

Optionally, in some specific implementations of the present disclosure, an end of the sleeve 31 is provided with a positioning groove 312, and the body portion 3211 and the first bent portion 3212 are provided in the positioning groove 312.

Specifically, as shown in FIG. 3, one end of the sleeve 31 close to the puncture blade tip 20 is provided with a positioning groove 312, and the body portion 3211 and the first bent portion 3212 of the limiting member 32 are provided in the positioning groove 312, so that the limiting member 32 can be assembled on the sleeve 31. This assembly structure can effectively ensure the assembly stability between the limiting member 32 and the sleeve 31.

According to one embodiment of the present disclosure, the limiting member 32 is an integrally formed metal sheet or metal wire.

That is, the connecting portion 321 and the limiting portion 322 of the limiting member 32 may be integrally formed, and they may be made of elastic metal wire or elastic metal sheet. The limiting member 32 of such a structure is not only convenient to manufacture, but also low in cost, and is suitable for popularization and use.

In some specific implementations of the present disclosure, the handle 10 is provided with a guide slot 11 extending along an axial direction thereof, the slide key 40 is movably provided in the guide slot 11, and a part of the slide key 40 passes through the guide slot 11 to be connected to the protective sleeve 30.

Specifically, as shown in FIG. 1, an outer wall surface of the handle 10 is provided with a guide slot 11 communicating with the inside of the handle 10, the slide key 40 is movably disposed in the guide slot 11, and a part of the slide key 40 passes through the guide slot 11 to be connected to the protective sleeve 30, so as to drive the protective sleeve 30 to move synchronously; another part of the slide key 40 may be exposed from the handle 10, or may be formed into a structure suitable for manual operation by a user, so that the user can drive the protective sleeve 30 to move by pushing the slide key 40 with one hand, thereby realizing the effect of pushing out the puncture cannula 50 with one hand.

Accordingly, by providing the slide key 40 on the handle 10, the process of pushing the puncture cannula 50 out of the puncture blade tip 20 can be realized by operating the slide key 40 with one hand, which further simplifies the operation mode, improves the surgical efficiency of the ophthalmic puncture device 100, and reduces surgical risks.

Although some specific embodiments of the present disclosure have been described in detail through examples, those skilled in the art should understand that the above examples are for illustration only and are not intended to limit the scope of the present disclosure. Those skilled in the art should understand that the above embodiments can be modified without departing from the scope and spirit of the present disclosure. The scope of the present disclosure is defined by the accompanying claims.

## Claims

1. An ophthalmic puncture device, **characterized by** comprising:
a handle;
a puncture blade tip provided at one end of the handle;
a protective sleeve provided inside the handle and movable between a first position and a second position along an axial direction of the handle, wherein the puncture blade tip is located inside the protective sleeve when the protective sleeve is located at the first position, and is exposed from the protective sleeve when the protective sleeve is located at the second position;
a slide key connected to the protective sleeve and movable along the axial direction of the handle to drive the protective sleeve; and
a puncture cannula detachably sleeved over the puncture blade tip, wherein the protective sleeve cooperates with the puncture cannula to push the puncture cannula out of the puncture blade tip when the protective sleeve moves from the second position to the first position.

2. The ophthalmic puncture device according to claim 1, **characterized in that** the protective sleeve comprises:
a sleeve movable along the axial direction of the handle, one end of the sleeve being provided with a mounting recess, a part of the slide key being connected to the mounting recess; and
a limiting member provided on the sleeve, the limiting member abutting against the puncture cannula when the protective sleeve is located at the second position.

3. The ophthalmic puncture device according to claim 2, **characterized in that** the limiting member comprises:
a connecting portion connected to the sleeve; and
a limiting portion movable relative to the connecting portion between a retracted state and an expanded state, wherein when the limiting portion is in the retracted state, the limiting portion avoids the puncture cannula, and the protective sleeve is movable from the first position to the second position; when the limiting portion is in the expanded state, the limiting portion is located on a movement path of the puncture cannula, and the limiting portion is separated from the puncture cannula or stops against the puncture cannula.

4. The ophthalmic puncture device according to claim 3, **characterized in that** the connecting portion is formed as an annular structure extending along a circumferential direction of the sleeve, and the limiting portion is provided on an inner circumference of the connecting portion and extends along a circumferential direction of the connecting portion.

5. The ophthalmic puncture device according to claim 3, **characterized in that** the limiting portion is formed as an elastic body provided on the connecting portion, and is deformable to switch between the retracted state and the expanded state.

6. The ophthalmic puncture device according to claim 5, **characterized in that** the limiting portion is formed as an elastic body extending obliquely relative to an axis of the puncture cannula, and in a direction in which the protective sleeve moves from the first position to the second position, the limiting portion gradually slopes away from the axis of the puncture cannula.

7. The ophthalmic puncture device according to claim 3, **characterized in that** the connecting portion comprises:
a body portion connected to the sleeve;
a first bent portion formed as a C-shaped structure, a first end of the first bent portion being connected to the body portion; and
a second bent portion formed as a C-shaped structure with an opening direction opposite to an opening direction of the first bent portion, a first end of the second bent portion being connected to a second end of the first bent portion, the limiting portion being connected to a second end of the second bent portion.

8. The ophthalmic puncture device according to claim 7, **characterized in that** an end of the sleeve is provided with a positioning groove where the body portion and the first bent portion are provided.

9. The ophthalmic puncture device according to claim 7, **characterized in that** the limiting member is an integrally formed metal sheet or metal wire.

10. The ophthalmic puncture device according to claim 1, **characterized in that** the handle is provided with a guide slot extending along an axial direction thereof, the slide key is movably provided in the guide slot, and a part of the slide key passes through the guide slot to be connected to the protective sleeve.
